# EUROPEAN PATENT APPLICATION

(11) **EP 1 466 565 A1**
(43) Date of publication of application: **13.10.2004**
(21) Application number: 03701112.9
(22) Date of filing: 17.01.2003
(51) Int. Cl.: A61B 17/56

(54) **METHOD OF TREATING OSTEOCHONDRITIS AND APPARATUS FOR TREATING OSTEOCHONDRITIS**

(30) Priority: 18.01.2002 JP 2002009832; 18.01.2002 JP 2002009833
(71) Applicant: TEIJIN LIMITED, Osaka-shi Osaka 541-0054 (JP); Nakamura, Toshitaka, Kitakyushu-shi, Fukuoka 807-0804 (JP); Yamaguchi, Hirotsugu, Kitakyushu-shi, Fukuoka 807-0804 (JP); Uchida, Soshi, Kitakyushu-shi, Fukuoka 807-0804 (JP); Tanaka, Masahiro, Kitakyushu-shi, Fukuoka 807-0804 (JP); Maekawa, Kazumichi, Kitakyushu-shi, Fukuoka 807-0871 (JP); Kanazawa, Yosuke, Shioya-gun, Tochigi 321-2523 (JP); Kashiwaguchi, Shinji, Tokushima-shi, Tokushima 770-0042 (JP)
(72) Inventor: Nakamura, Toshitaka, c/o Univ. of Occupational and, Kitakyushu-shi, Fukuoka 807-0804 (JP); Yamaguchi, Hirotsugu c/o Univ. of Occupational and, Kitakyushu-shi, Fukuoka 807-080 (JP); Uchida, Soshi, c/o Univ. of Occupational and, Kitakyushu-shi, Fukuoka 807-0804 (JP); Tanaka, Masahiro, c/o Univ. of Occupational and, Kitakyushu-shi, Fukuoka 807-0804 (JP); Maekawa, Kazumichi, c/o Maekawa Orthopedics, Kitakyushu-shi, Fukuoka 807-0871 (JP); Kanazawa, Yosuke, Rosai Hospital For Silicosis, Shioya-gun, Tochigi 321-2523 (JP); Kashiwaguchi, Shinji, The University of Tokushima, Tokushima-shi, Tokushima 770-0042 (JP)
(74) Representative: HOFFMANN - EITLE
(86) International application number: PCT/JP2003/000365
(87) International publication number: WO 2003/061492

(57) **Abstract**

A method for treating osteochondrosis comprising the irradiation of a joint of a patient of osteochondrosis with ultrasound is provided, as a method enabling the shortening of a period of a conservative treatment such as the limitation of movement, the treatment of a case which does not respond to a conservative treatment and the alleviation of pain by accelerating the repair of a damaged site of osteochondrosis. As an apparatus therefor, an apparatus for treating osteochondrosis comprising an ultrasound treatment head module having a built-in ultrasound transducer and applying ultrasound on a joint caput site of a patient of osteochondrosis, a control means for controlling the ultrasound, and a joint fixing means having functions of fixing the joint at a specified angle in the patient of osteochondrosis and simultaneously fixing the ultrasound treatment head module on the joint site is provided.

## Description

### Technical Field

The present invention relates to a method for treating osteochondrosis and an apparatus therefor. More particularly, it relates to a method for shortening a treatment period or for avoiding an invasive operation in osteochondrosis such as Osgood-Schlatter disease, sport elbow (baseball elbow, tennis elbow etc.) or Kienbock's disease by alleviating pain and accelerating the repair of damaged sites by applying ultrasound, and an apparatus therefor.

### Background Art

It is considered empirically right that mechanical stimulation is effective for healing bone fractures, and a number of researchers carried out experimental or clinical studies on this subject. Kenwright et al. of Oxford University reported that bone union was promoted significantly by repeatedly applying axial compressive stimulation directly on the fractured site by using an external skeletal fixture (10 minutes per day, 0.5 Hz (=600 times), micromovement of 1 mm in the major axis direction of the bone) (J. Bone Joint Sur. Br 73 (4): 654-659, 1991).

Duarte considered that osteogenesis was accelerated noninvasively when mechanical stimulation with ultrasound was applied on bone tissue, and he studied on the conditions of ultrasound application suitable for the treatment by using animal models from 1980 to 1985. A part of the studies indicated that a normal bone repair process was accelerated as the result of the irradiation of rabbit osteotomy model with low-intensity pulsed ultrasound in 1983 (Arch Orthop Trauma Surg, 101: 153-159, 1983).

Further, Xavier and Durate reported that they applied low-intensity pulsed ultrasound on 27 patients in 1983, resulting in the acceleration of the normal fracture healing process and the induction of the repair of non-union (Rev Brasil Ortop, 18: 73-80, 1983).

Thereafter, Exogen Inc. in USA, which had been licensed from Duarte for his basic patent (United State Patent No. 4,530,360), developed an ultrasound-accelerated fracture healing device, SAFHS™, and demonstrated healing-acceleration effects on fractures of the tibial diaphysis (Heckman et al., J. Bone and Joint Surg., 76A: 25-34, 1994), on fractures of the distal radius (Kristiansen et al., J. Bone and Joint Surg., 79A:961-973, 1997) and so on in clinical trials.

It was demonstrated in an animal study using dogs that when ultrasound was applied on a fractured site using SAFHS™, the local blood flow was increased (Goldberg et al., North American Radiological Society Meeting, November, 1997). According to studies conducted in the US and Europe, ultrasound was effective on intractable fractures that were due to insufficient blood flow. Accordingly, it is considered that "the acceleration of blood flow" is an important mechanism. Further, Bolander and Ryaby et al., suggested that ultrasound from SAFHS™ had direct action on osteogenesis and bone resorption, noncollagen protein synthesis, and the expression of collagen phenotype.

As shown above, SAFHS™ is considered to be effective on the treatment of osteochondrosis since it accelerates osteogenic reaction of an adult.

Osteochondrosis is ischemic osteonecrosis occurred on an epiphyseal ossification center (the secondary ossification center) of a long bone, or the primary ossification center or an apophysis of a short bone during growth process. It is classified into the following three groups depending on the kinds of external force applied on the epiphyseal ossification center or the apophysis; (1) a crushed type (Kienbock's disease, Perthes disease and the like), (2) a sheared type (sport elbow such as baseball elbow and tennis elbow, and the like), and (3) a pulled type (Osgood-Schlatter disease and the like).

For the treatment of osteochondrosis, a conservative treatment such as limitation of movement is mainly adopted. But there are some cases wherein no effect is observed during the conservative treatment, and a therapy with a surgical operation is used in such cases.

Further, bone diseases such as osteochondrosis are always accompanied by pain. The treatment of pain is mainly performed by a medicinal treatment through oral administration, intravenous administration or continuous subcutaneous infusion. However, these treatments have troubles such as side effects of analgesics, the existence of patients having resistance to the drugs and difficulties in the control of the amounts of the drugs. Further, a nerve block therapy could be implemented as a surgical treatment. However, sufficient attention must be paid on a risk accompanied by the surgery adding to the above mentioned troubles. As a treatment without medicine, there are transcutaneous electrical nerve stimulation (TENS), transcutaneous electrical acupuncture stimulation (TEAS), silver spike point therapy (SSP therapy) and so on, but the effects for these methods are not determined yet. Further, as an invasive treatment, there is also spinal cord stimulation electrode-embedding, but this treatment has a risk accompanied by the surgical operation and gives a burden on the patient in pain control at home.

### Disclosure of the Invention

The present invention accelerates the repair of damaged sites in osteochondrosis, and thereby the period of a conservative therapy is shortened, and even the cases which do not respond to a conservative therapy can be treated, and the avoidance of a surgical operation becomes possible. Further, it provides a new treatment method that is different from a conventional method for treating pain accompanied by osteochondrosis, and reduces the burden of the patient and improves QOL (the quality of life) of the patient.

The inventors of the present invention pursued zealous studies on such problems, and they found that the application of mechanical and dynamic stimulation by the irradiation of ultrasound to a damaged site in osteochondrosis, accelerates the repair of the damaged site in osteochondrosis, and exhibits pain alleviation effect. Thus, they reached the present invention.

The present invention provides a device for treating osteochondrosis comprising the following units: an ultrasound treatment head module which has a built-in ultrasound transducer and applies ultrasound on a joint caput site of a patient of osteochondrosis; a control means for controlling the ultrasound; and a joint fixing means, which has functions of fixing the joint of the patient of osteochondrosis at a specified angle, and simultaneously fixing the ultrasound treatment head module on the joint site.

Further, the present invention provides an apparatus for treating osteochondrosis characterized in that the control means controls pulsed ultrasound from the ultrasound transducer, and it controls the ultrasound having a frequency of 20 kHz to 10 MHz, a burst width of 10 µsec to 1 msec, a repetition rate of 5 Hz to 10 kHz, and an ultrasound intensity of 5-75 mW/cm², preferably, the frequency of 1.5 MHz, the burst width of 200 µsec, the repetition rate of 1.0 kHz and the ultrasound intensity of 30 mW/cm².

Furthermore, the present invention provides an apparatus for treating osteochondrosis to be applied on the joint site of a patient of Osgood-Schlatter disease or sport elbow.

Further, the present invention provides a method for treating osteochondrosis characterized in that a joint of a patient of osteochondrosis is irradiated with ultrasound.

Further, the present invention provides a method for treating osteochondrosis characterized in that the pulsed ultrasound having a frequency of 20 kHz to 10 MHz, a burst width of 10 µsec to 1 msec, a repetition rate of 5 Hz to 10 kHz, and an ultrasound intensity of 5-75 mW/cm², preferably, the frequency of 1.5 MHz, the burst width of 200 µsec, the repetition rate of 1.0 kHz and the ultrasound intensity of 30 mW/cm² is applied.

Furthermore, the present invention provides a method for treating osteochondrosis characterized in that the ultrasound is applied on a joint site of the patient of osteochondrosis by means of a joint fixing means that has functions of fixing the joint site at a specified angle and simultaneously fixing an ultrasound treatment head module having a built-in ultrasound transducer on the joint site.

The present invention is applied to a method for treating especially Osgood-Schlatter disease or sport elbow, and to a method for alleviating pain accompanied by osteochondrosis by using the above-mentioned treating method for osteochondrosis.

### Brief Description of Drawings

Figure 1 is a sketch drawing of an apparatus for treating osteochondrosis of the present invention.
Figure 2 is a block diagram exhibiting the construction of the main operating unit of the apparatus for treating osteochondrosis of the present invention.
Figure 3 is a block diagram exhibiting the construction of an ultrasound treatment head module of the apparatus for treating osteochondrosis of the present invention.

### Best Mode for Carrying Out the Invention

The present invention provides a treatment method for osteochondrosis characterized in that the repair of a damaged site of osteochondrosis is accelerated by the application of a mechanical/dynamic stimulation (mechanical stress) having a certain pattern on the damaged site by irradiating the damaged site with ultrasound, and an apparatus for treating osteochondrosis therefor.

Hereafter, examples of embodiments of an apparatus for treating osteochondrosis of the present invention are shown. The apparatus for treating osteochondrosis of the present invention comprises an ultrasound treatment head module which has a built-in ultrasound transducer and applies ultrasound on a joint caput site of a patient of osteochondrosis, a control means for controlling the ultrasound, and a joint fixing means that has functions of fixing the joint of the patient of osteochondrosis at a specified angle and simultaneously fixing the ultrasound treatment head module on the joint site.

The ultrasound transducer is made of a piezoelectric ceramic. Electric signals produced by an ultrasonic wave synthesizing apparatus are given to the piezoelectric ceramic of the transducer, and the transducer generates vibration (ultrasound) in the longitudinal direction on its surface by using the characteristics that microstrains are generated when voltage is applied.

The ultrasound to be used is low-intensity pulsed ultrasound, and the ultrasound having a frequency of 20 kHz to10 MHz, a burst width of 10 µsec to 1 msec, a repetition rate of 5 Hz to 10 kHz and an ultrasound intensity of 5-75 mW/cm² is expected to be effective for the treatment. Especially, the ultrasound having the frequency of 1.5 MHz, the burst width of 200 µsec, the repetition rate of 1.0 kHz and the ultrasound intensity of 30 mW/cm² is preferable.

The control means is provided with an ultrasonic wave synthesizing function with which the ultrasound radiated from the ultrasound transducer is synthesized, an intensity controlling function, a storage arithmetic means and an on-off controlling function for electricity. These functions and means may be integrated with the ultrasound treatment head module and built in the head module, or may be divided into the ultrasound treatment head module and the main operating unit.

### Examples

### Example 1

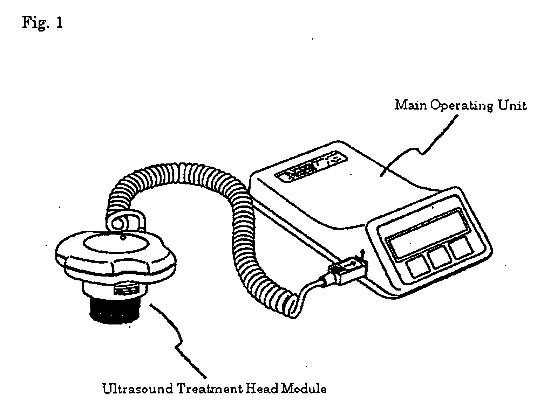

Hereafter, an example in which an apparatus for treating osteochondrosis of the present invention is divided into a main operating unit and an ultrasound treatment head module is explained by using figures. The apparatus for treating osteochondrosis of the present invention comprises the main operating unit and the ultrasound treatment head module in its appearance as shown by Figure 1.

### 1. Main operating unit

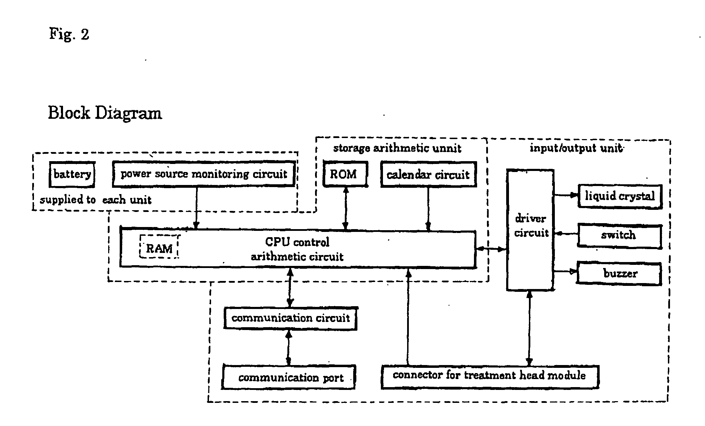

The main operating unit is constituted with a power source, a storage arithmetic unit and an input/output unit as shown by Figure 2.
(1) Power source: a high capacity-type non-chargeable lithium battery is used. It has no power switch, the power is put on by the operation of the front panel, and the power is automatically turned off after the completion of a 20 minute-treatment. In order to prevent the output of ultrasound by miss operation, it is required to press the start button twice for outputting the ultrasound. The treatment head is energized from the same battery.
(2) Storage arithmetic unit: a CPU (central processing unit) is mounted on a printed board, and the unit not only manages a treatment length but also monitors the movement and the state of the mounting of the treatment head, and also performs a self-diagnosis. Further, the therapy records of patients are kept on a backup memory, and they can be taken out as required.
(3) Input/output unit: the controls of the treatment head module and the power supply to it are performed via an operation switch, a liquid crystal display board, a buzzer and an interconnection cable. To the treatment head module, control signals having a pulsed ultrasound burst width of 200 Hz and a repetition rate of 1 kHz are sent. Further, trouble signals are returned from the head module.

### 2. Ultrasound treatment head module

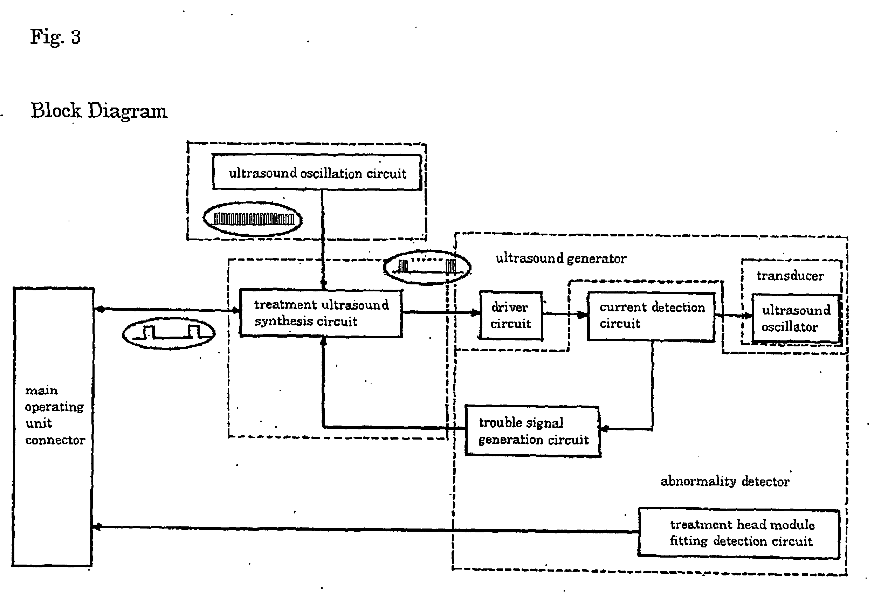

The ultrasound treatment head module is constituted with an oscillator, an ultrasonic wave synthesizing unit, an ultrasound generator and a malfunction detector as shown in Figure 3. The interconnection cable to the main operating unit is an integrated type.
(1) Oscillator: ultrasound signal of 1.5 MHz is generated.
(2) Ultrasonic wave synthesizing unit: control signals from the main operating unit and an ultrasound signal of 1.5 MHz are combined to produce treatment ultrasound, that is, electric signals of waveform having signal properties of a frequency of 1.5 MHz, an ultrasound intensity of 30 mW/cm², a burst width of 200 µsec and a repetition rate of 1 kHz.
(3) Ultrasound generator: a piezoelectric ceramic has characteristics in which voltage application generates microstrains. By using the properties, electric signals produced by an ultrasonic wave synthesizing unit are given to the piezoelectric ceramic inside the transducer, and vibration (ultrasound) in its longitudinal direction is produced on the surface of the transducer. The vibration is used for the treatment. On the backside of the piezoelectric ceramic, an ultrasound absorber is adhered to prevent the leakage of the ultrasound.
(4) Malfunction detector: the defective fixing of the treatment head module and the shortage of ultrasound transmission gel are detected, and they are sent to the main operating unit, and informed to the patient by a liquid crystal display and an alarm. The detection of defective fixing between the head module and the fixture is performed based on the physical contact of fixing parts, that is, it is performed by judging the existence or absence of electric conductivity. The shortage of the gel is detected by the change of resonance state of an ultrasound oscillator due to the shortage of the gel, this being judged by the increase in the driving current from the normal level.

### 3. Method for treatment

The ultrasound treatment head module is placed on the skin of the joint site, and ultrasound is applied for 20 min once a day on an arm joint site or knee joint site of a patient of osteochondrosis. For treatment, the fixture with which the arm or knee is fixed usually in a state where the joint is bent at a roughly right angle, is mounted, and the ultrasound treatment head module is attached on the fixture. A joint fixture described in a pamphlet of WO 00/47142 can be used as the fixture.

### Example 2

The effect of a treatment on an Osgood-Schlatter disease patient was confirmed by using the treating apparatus for osteochondrosis described in Example 1.

Purpose: Osgood-Schlatter disease (OSD), which is often diagnosed and treated as a damage associated with sports during a growth period, has a relatively good prognosis, but in some cases, aggravation and remission are repeated, and the treatment is difficult. By using low-intensity pulsed ultrasound we treated OSD patients who did not respond to a conservative treatment, and confirmed the effects of the treatments.

Targets: OSD patients who visited a hospital from 1999 to 2001 and did not respond to a preservative treatment for more than 6 months were treated with an apparatus for treating osteochondrosis of the present invention. Subjects were 9 cases and 12 knees comprising 8 cases and 11 knees of male, and one knee of female. They were aged from 11 to 15, and 13.1 year old in average. The period from the onset of the disease to the start of the treatment with the apparatus for treating osteochondrosis of the present invention ranged from 6 months to 3.5 years, and it was 1.4 years in average. The period of the treatment ranged from 2 months to 6 months, and it was 3.6 months in average.

Results: pains disappeared or eased in 11 knees out of 12 knees, and these patients were able to return to sports. Radiologically, bridging was observed at an avulsion part of the tibial tuborosity in 8 knees out of 12 knees, but 3 knees out of the 8 knees exhibited the expansion of a radiolucent region along epiphysis. The other 3 knees out of 12 knees had no bridging radiologically but exhibited the expansion of radiolucent region along epiphysis, and the progress of OSD was suspected radiologically in these knees. One knee exhibited no change radiologically.

Conclusion: the apparatus for treating osteochondrosis of the present invention exhibited therapeutic effect on the cases of OSD radiologically. Further, even in the cases wherein no therapeutic effect was observed radiologically, the tendency of the improvement of clinical symptoms such as the disappearance or easing of pain was observed.

### Example 3

The therapeutic effect on a patient of chondral damage of the little head of humerus, baseball elbow, was confirmed by using the apparatus for treating osteochondrosis described in Example 1.

Object: Chondral damage of the little head of humerus is an intractable disease which occurs on a baseball player in the growing period. By using an apparatus for treating osteochondrosis of the present invention, low-intensity pulsed ultrasound was applied on a patient who had been treated by a conservative treatment or by a fixation of detached cartilage, and the therapeutic effect was examined.

Method: The number of the treated patients were 10. They were from 10.8 to 14.3 year old, and 12.4 year old in average. Regarding the stage of damage, 5 cases were in the initial stage, 4 cases were in the progressive stage, and one case was in the terminal stage. Ultrasound from a SAFHS™ was applied for 20 min everyday on the central part of the humeroradial joint in a state where the elbow joint is bent at 90 degree.

Result: Repair, incomplete repair and non-repair were observed in 4 cases, 2 cases and 1 case, respectively. Three cases were under follow-up. In the repair group, time needed for the repair was 10 months in average for 3 cases excluding one case in which an external wound of the elbow occurred as a complication during the treatment. This was almost same as an average treatment period in a conservative treatment in which only throwing was inhibited, and no clear shortening of the treatment period was observed. However, it is meaningful that in one case in which therapeutic mechanism probably stopped after one-year conservative treatment, repair was observed with an 8-month treatment with SAFHS™. In the incomplete repair group, one case showed tendency of repair, but the patient strongly desired to undergo a surgical operation, so that the treatment with SAFHS™ was stopped. The other one case was treated with SAFHS™ after the fixation of detached cartilage, and a partial defect in bone was formed. In the non-repair case, whose treatment with SAFHS™ was initiated in the terminal stage and which had a loose body, the loose body was not united after all. One of the 3 follow-up cases developed the tendency of repair after 4 months of the treatment.

### Example 4

Pain alleviation effect on Osgood-Schlatter disease patients was confirmed by using an apparatus for treating osteochondrosis described in Example 1.

Targets: Osgood-Schlatter disease patients who visited a hospital from 1999 to 2001 and did not respond to a preservative treatment for more than 6 months were treated with the apparatus for treating osteochondrosis. Subjects were 9 cases and 12 knees comprising 8 cases and 11 knees of male, and one knee of female. They were aged from 11 to 15, and 13.1 year old in average. The period from the onset of the disease to the start of the treatment with ultrasound ranged from 6 months to 3.5 years, and it was 1.4 years in average. The period of the treatment ranged from 2 months to 6 months, and it was 3.6 months in average.

Results: The disappearance or easing of pain was observed in 11 knees out of 12 knees, and the patients could return to sports.

### The effect of the Invention

As mentioned above, the use of the method and the apparatus for treating osteochondrosis of the present invention accelerates the repair of a damaged part of osteochondrosis, shortens the period of a conservative treatment, and enables the treatment of a case which do not respond to a conservative treatment and the avoidance of a surgical operation. Further, it can alleviate pain, and it is effective for improving QOL of a patient.

## Claims

1. An apparatus for treating osteochondrosis comprising an ultrasound treatment head module which has a built-in ultrasound transducer and applies ultrasound on a joint caput site of a patient of osteochondrosis, a control means for controlling the ultrasound, and a joint fixing means having functions of fixing the joint at a specified angle in the patient of osteochondrosis and simultaneously fixing the ultrasound treatment head module on the joint site.

2. An apparatus for treating osteochondrosis described in claim 1 **characterized in that** the control means outputs and controls pulsed ultrasound from the ultrasound transducer.

3. An apparatus for treating osteochondrosis described in claim 2 **characterized in that** the control means outputs and controls ultrasound having a frequency of 20 kHz to 10 MHz, a burst width of 10 µsec to 1 msec, a repetition rate of 5 Hz to 10 kHz, and an ultrasound intensity of 5 to 75 mW/cm².

4. An apparatus for treating osteochondrosis described in claim 3 **characterized in that** the control means outputs and controls ultrasound having the frequency of 1.5 kHz, the burst width of 200 µsec, the repetition rate of 1.0 kHz, and the ultrasound intensity of 30 mW/cm².

5. An apparatus for treating osteochondrosis described in any one of Claims 1 to 4 **characterized in that** the apparatus is applied on a joint site of a patient of Osgood-Schlatter disease or a sport elbow.

6. A method for treating osteochondrosis **characterized in that** a joint of a patient of osteochondrosis is irradiated with ultrasound.

7. A method for treating osteochondrosis described in claim 6 **characterized in that** the pulsed ultrasound having a frequency of 20 kHz to 10 MHz, a burst width of 10 µsec to 1 msec, a repetition rate of 5 Hz to 10 kHz, and an ultrasound intensity of 5 to 75 mW/cm² is applied.

8. A method for treating osteochondrosis described in claim 6 **characterized in that** the pulsed ultrasound having the frequency of 1.5 kHz, the burst width of 200 µsec, the repetition rate of 1.0 kHz, and the ultrasound intensity of 30 mW/cm² is applied.

9. A method for treating osteochondrosis described in Claim 6 **characterized in that** the ultrasound is applied by means of a joint fixing means having functions of fixing the joint at a specified angle in the patient of osteochondrosis and simultaneously fixing an ultrasound head module having a built-in ultrasound transducer on the joint site.

10. A method for treating Osgood-Schlatter disease or a sport elbow by using a method for treating osteochondrosis described in any one of Claims 6 to 9.

11. A method for treating pain by using a method for treating osteochondrosis described in any one of Claims 6 to 9.
